# EUROPEAN PATENT APPLICATION

(11) **EP 4 570 325 A1**
(43) Date of publication of application: **18.06.2025**
(21) Application number: 23216973.0
(22) Date of filing: 15.12.2023
(51) Int. Cl.: A61Q 11/00, A61K 8/19, A61K 8/26

(54) **NOVEL DENTIFRICE COMPOSITION**

(71) Applicant: Haleon UK IP Limited, Weybridge, Surrey KT13 0NY (GB)
(72) Inventor: The designation of the inventor has not yet been filed
(74) Representative: Haleon Patent Department

(57) **Abstract**

The invention relates to a sodium bicarbonate based toothpaste with a novel abrasive composition to provide superior polishing performance.

## Description

### Field of the invention

The invention relates to a dentifrice composition with superior cleaning and polishing performance. The dentifrice is a high bicarbonate formula.

### Background to the invention

The use of sodium bicarbonate in dentifrices products is well known. One example is the commercial product Paradontax/Corsodyl which contains 67 % (w/w) of sodium bicarbonate.

Any dentifrice, in particular a sodium bicarbonate dentifrice must be acceptable from a consumer standpoint and demonstrate, for example, acceptable taste, consistency and adequate foaming on brushing of teeth. Any such composition must also be stable and not suffer from syneresis, which is the contraction of a gel accompanied by the separating out of liquid.

WO2019/057809 disclosed the difficulties in working with high bicarbonate toothpastes and is herein incorporated by reference.

Sodium bicarbonate pastes offer great cleaning performance and gum health benefits. Sodium bicarbonate based pastes are not well known for having a good polishing effect.

Polishing is not just about appearance, although as it imparts a smooth and finished look to teeth, polished teeth do tend to look and feel good. Polishing can really help to clear the tooth surface from all plaque and tartar.

### Statements of Invention

In its broadest aspect the invention encompasses a dentifrice composition comprising at least 50 % by weight of sodium bicarbonate and alumina.

In another embodiment the dentifrice composition comprises alumina between 0.5 % and 3 % by weight.

In another embodiment the dentifrice composition comprises alumina between 1.0 % and 1.5 % by weight.

In another embodiment the dentifrice composition the alumina has a d₅₀ particle size of between about 4µm and 10 µm

In another embodiment the dentifrice composition according to any one of claims 1 to 4 wherein the sodium bicarbonate is present in an amount from about 55 % to about 75 % by weight of the composition.

In another embodiment the dentifrice composition comprises between about 10 % to about 20 % by weight of water.

In another embodiment the dentifrice composition according to any one of the previous claims wherein the composition further comprises a humectant between about 5 % and about 10% by weight.

In another embodiment the dentifrice composition comprises at least one surfactant.

In another embodiment the dentifrice composition according to any one of claims 1 to 8 additionally comprising a fluoride source, a desensitizing agent, an anti-plaque agent, an anti-calculus agent, a whitening agent, an oral malodour agent, an anti-inflammatory agent, an antimicrobial agent, an anti-oxidant, an anti-fungal agent, a wound healing agent or a mixture of at least two thereof.

In another embodiment the dentifrice composition comprises a sweetener, a colourant, a flavouring, a pH modifier, a thickening gum or a mixture of at least two thereof.

In another embodiment the dentifrice composition comprises:
Sodium bicarbonate between about 60 % and about 70 % by weight
Water between about 12 % and about 15 % by weight
Humectant between about 5 % and about 10 % by weight
At least one surfactant between about 0.5 % and about 3% by weight
Alumina between about 0.5 % and about 3.0 % by weight.

In another embodiment the dentifrice composition further comprises an additional abrasive.

In another embodiment the dentifrice composition further comprises a spherical silica.

In another embodiment the dentifrice composition comprises:
Sodium bicarbonate between about 62 % to about 68 % by weight;
Water between about 12 % and about 15 % by weight;
Glycerol between about 5 % and about 10 % by weight;
At least one surfactant between about 1.0 % and about 2.0 % by weight
Alumina between about 1.0 % and about 1.5% by weight; and
A source of fluoride ions.

### Detailed description of the invention

The applicants have found that the combination of alumina with high levels of bicarbonate in a formulation provides a dentifrice with high level of gloss on teeth post brushing.

Gloss is an attractive feature in a dentifrice. Consumers prefer to see a shiny surface to their teeth once completing brushing.

Suitably the sodium bicarbonate is present in an amount from 55% to 90% w/w, preferably from 60% to 80% w/w, more preferably from 61% to 70% w/w; e.g. from 62% to 68% w/w of the dentifrice composition.

Further the dentifrice composition of the present invention comprises alumina.

Aluminium oxide (or aluminium(III) oxide) is a chemical compound of aluminium and oxygen with the chemical formula Al₂O₃. It is the most commonly occurring of several aluminium oxides, and specifically identified as aluminium oxide. It is commonly called alumina and may also be called aloxide, aloxite, or alundum in various forms and applications.

It occurs naturally in its crystalline polymorphic phase α-Al₂O₃ as the mineral corundum, varieties of which form the precious gemstones ruby and sapphire. Al₂O₃ is significant in its use to produce aluminium metal, as an abrasive owing to its hardness, and as a refractory material owing to its high melting point.

Preferably the alumina is used in the present invention between about 0.5 % and about 5 % by weight, preferably about 0.6 % to about 3 % by weight, more preferably between about about 0.8 % and about 2.5% by weight, more preferably between about 1.0 % and about 2.0 %.

In preferred embodiment the alumina comprises between about 1.0 % and about 1.5 % by weight of the dentifrice.

Preferably the alumina added to the dentifrices of the present invention has a d50 particle size of between about 4 µm and about 10 µm.

A particularly preferred alumina suitable for use in the dentifrice compositions of the present invention is P10 Feinst supplied by the Almatis chemical company.

The dentifrice compositions of the present invention may comprise may further ingredients suitable for use in dentifrice compositions.

The dentifrice composition accord to the present invention may comprise a humectant.

Non-limiting examples of suitable humectants for use in compositions of the invention include glycerin, xylitol, sorbitol, propylene glycol or polyethylene glycol, or mixtures of at least two thereof;

A particularly preferred humectant is glycerin.

The humectant may comprise between about 1% and about 20% by weight of the dentifrice composition, more preferably between about 2.5 % and about 15 % by weight of the dentifrice composition and most preferably between about 5 % and about 10% by weight of the dentifrice composition.

The dentifrice compositions of the present invention may comprise water.

The water may comprise between about 1% and about 20% by weight of the dentifrice composition, more preferably between about 5 % and about 17.5 % by weight of the dentifrice composition and most preferably between about 10 % and about 15 % by weight of the dentifrice composition.

The dentifrice composition according to the invention may comprise at least one surfactant.

The at least one surfactant may comprise any surfactant known in the oral care art.

The dentifrice compositions of the present invention may comprise at least about 0.1 % by weight of surfactant, preferably at least about 0.5% by weight surfactant, more preferably at least about 1.0% by weight surfactant.

The dentifrice compositions of the present invention may comprise a single surfactant. The dentifrice compositions of the present invention may comprise a surfactant system that comprises two or more surfactants.

The dentifrice compositions may comprise a total surfactant content between about 0.1 % and about 7.5 % by weight, more preferably between about 0.5 % and about 5 % by weight and most preferably between about 1.0 % and 3.0 % by weight.

An example of a suitable surfactant system comprises a betaine surfactant and sodium lauryl sulphate surfactant present in a total amount of about 2 % to about 4 % by weight of the composition, preferably in a total amount of about 2 % to about 3 % by weight of the composition.

Structurally, the betaine compounds contain an anionic functional group such as a carboxylate functional group and a cationic functional group such as quaternary nitrogen functional group separated by a methylene moiety. They include n-alkyl betaines such as cetyl betaine and behenyl betaine, and n-alkylamido betaines such as cocoamidopropyl betaine. In one embodiment the betaine is cocoamidopropyl betaine, commercially available from Evonik Industries AG under the trade name TEGO Betain C60.

Suitably the betaine may be present in an amount ranging from about 0.3 % to about 2 % by weight, for example from about 0.4% to about 1.5 % by weight and preferably from about 0.5% to about 0.7% by weight of the composition. A suitable betaine is TEGO Betain C60 which may be bought and formulated as a 47 % aqueous solution and the figures quoted in brackets in the Examples are the active matter level of cocoamidopropyl betaine in the 47 % solution.

Suitably the sodium lauryl sulphate (SLS) surfactant may be present in an amount from about 0.5 % to 3.5 % by weight, preferably in an amount from about 1.0 % to about 3.0 % w/w, more preferably in an amount from about 1.5 % to about 2.5 % w/w and most preferably in an amount from 1.75 % to 2.25 % by weight.

Other suitable surfactants include taurates.

The invention is not limited to compositions comprising particular types or amounts of surfactants. The skilled person will be aware of suitable further surfactants that can be used with the compositions of the present invention.

In addition to the above ingredients, compositions of the present invention may comprise a fluoride source, a desensitising agent, an anti-plaque agent, an anti-calculus agent, a whitening agent, an oral malodour agent, an anti-inflammatory agent, e.g. monoammonium glycyrrhizinate (MAG), an antimicrobial e.g. isopropylmethylphenol (IPMP), an anti-oxidant, an anti-fungal agent, a wound healing agent, e.g. hyaluronic acid, or a mixture of at least two thereof. Such agents may be included at levels to provide the desired therapeutic effect.

Suitable sources of fluoride ions for use in the compositions of the present invention include an alkali metal fluoride such as sodium fluoride, an alkali metal monofluorophosphate such a sodium monofluorophosphate, stannous fluoride, or an amine fluoride in an amount to provide from 25ppm to 3500 ppm of fluoride ions, preferably from 100 ppm to 1500 ppm. A typical fluoride source is sodium fluoride, for example the composition may contain 0.1% to 0.5 % by weight of sodium fluoride, e.g. 0.204 % by weight (equating to 927ppm of fluoride ions), 0.2542 % by weight (equating to 1150 ppm of fluoride ions) or 0.315 % by weight (equating to 1426 ppm of fluoride ions).

Such fluoride ions help promote the remineralisation of teeth and can increase the acid resistance of dental hard tissues for combating caries, dental erosion (i.e. acid wear) and/or tooth wear.

Sodium bicarbonate will provide abrasive properties to the composition however an additional abrasive may also be present, e.g. a silica that has high cleaning properties. Examples of high cleaning silica abrasives include those marketed as Zeodent 124, Tixosil 63, Sorbosil AC39, Sorbosil AC43 and Sorbosil AC35 and may be present in suitable amounts for example up to 20 %, such as from about 5 % to about 10 %, preferably from about 1.5 % to about 7 %, e.g. 2 % by weight of the total composition.

Compositions of the present invention will contain additional formulating agents such as flavouring agents, sweetening agents, opacifying or colouring agents and preservatives, selected from those conventionally used in an oral hygiene composition art for such purposes.

**Base formulation of the invention comprises:**
Sodium bicarbonate >50 % by weight
At least one surfactant between about 0.5 % and about 5 %; and
Alumina between about 0.5 % and about 3.0 % by weight.

**A particularly preferred dentifrice composition of the invention comprises**
sodium bicarbonate between about 60 % and about 70 % by weight
water between about 12 % and about 25 % by weight
A humectant between about 5 % and about 10 % by weight
At least one surfactant between about 0.5 % and about 3 % by weight and
Alumina between about 0.5 % and about 3.0 % by weight.

**An even more preferred dentifrice composition of the invention comprises:**
Sodium bicarbonate between about 62 % and about 68 % by weight;
Water between about 15 % and about 22% by weight;
Glycerol between about 5 % and about 10 % by weight;
At least one surfactant between about 1.0 % and about 2.0 % by weight
Alumina between about 1.0 and about 1.5% by weight; and
A source of fluoride ions.

### Results

### Base Parodontax (67% bicarbonate) formula:

| **Ingredient** | **% by weight** |
|---|---|
| Sodium Bicarbonate | 67.3 |
| Water | 20.8 |
| Glycerin | 7.5 |
| Surfactant | 1.5 |
| Sodium Fluoride | 0.3 |
| Minor ingredients | 2.6 |

The compositions on the following table were tested via polishing protocol:

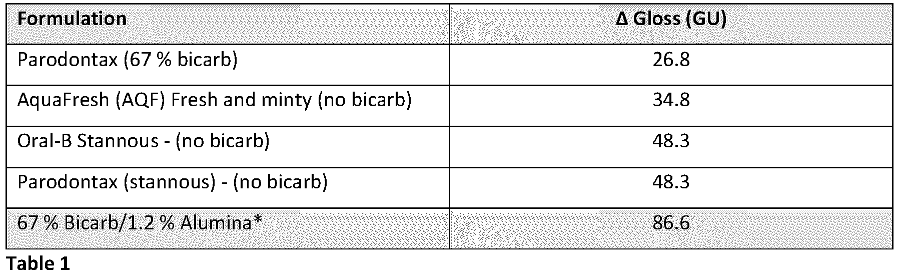

The four commercial formulas had a Δ Gloss (GU) ranging from 26.8 to 48.3.

The formulation according to the present invention (*modified commercial Parodontax sample) with addition of 1.2% by weight of almumina (Feinst P 10 by Almatis Ltd) had a Δ Gloss (GU) score of 86.6. This represents a massive improvement over the base formulation.

The base high level sodium bicarbonate formulations have always had a lower polishing score than other commercial pastes. The addition of alumina to these formulations provides superb polishing performance.

The Aquafresh formula was also then prepared with the same 1.2% alumina and tested in the same gloss testing methodology. The Δ Gloss (GU) improved by 20 units to 55.

This demonstrates there is a synergistic effect between the bicarbonate and the alumina that gives generates an unexpectedly great polishing performance.

Further samples were prepared to explore the scope of the invention. (The addition of ingredients balanced by concomitant reduction in water level of base formula)

Even 0.5% by weight of alumina was found to dramatically improve the polishing score of high barbarb pastes. The polishing performance improved even relative to compositions with additional abrasive/cleaning silicas such as AC 43 from PQ Corp and NP-30 from Sunsphere.

The addition of these silicas with the alumina also did not negatively impact the improved polishing performance.

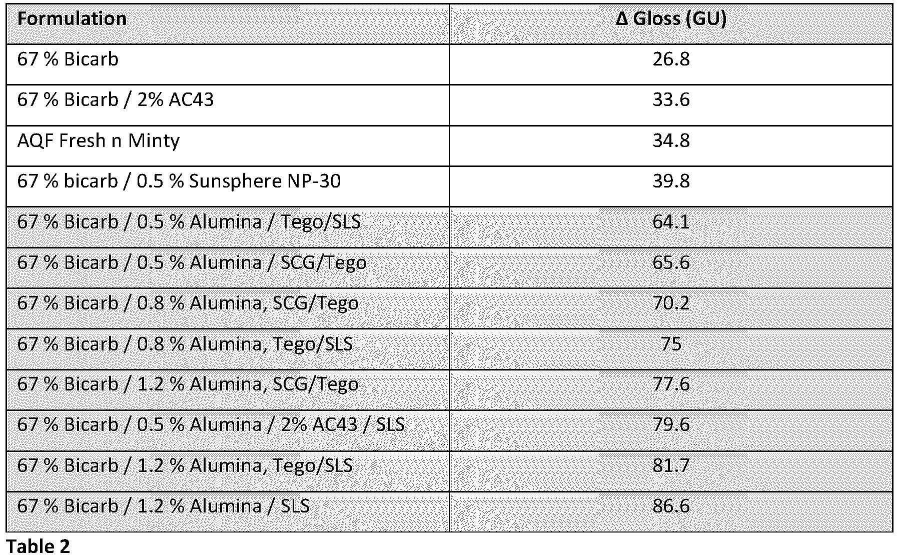

Such high levels of polishing required confirmation that the enamel would not be negatively impacted by the combination of the bicarbonate and alumina.

Radioactive dentine analysis (Table 3) showed that the addition of alumina was having only a very limited effect on dentine erosion.

**Table 3**

| **Formulation - (67% Bicarb)** | **RDA** | **SD** |
|---|---|---|
| 0.5 % Alumina, SCG/Tego | 69.55 | 4.19 |
| 0.8 % Alumina, SCG/Tego | 70.03 | 3.94 |
| 1.2 % Alumina, SCG/Tego | 66.38 | 3.92 |
| 0.5 % Alumina, Tego/SLS | 72.06 | 3.41 |
| 0.8 % Alumina, Tego/SLS | 66.17 | 3.48 |
| 1.2 % Alumina, Tego/SLS | 75.51 | 3.55 |

The alumina containing dentifrices of the present invention were also demonstrating improved cleaning over the base formulations. In pellicle cleaning tests the formulations were providing scores equivalent to known silica abrasives (AC 43 from PQ corp and spherical silica NP-30 from Sunsphere).

Table 4 details the PCR scores for a range of different pastes. The addition of all abrasives show an improvement over the base formulation.

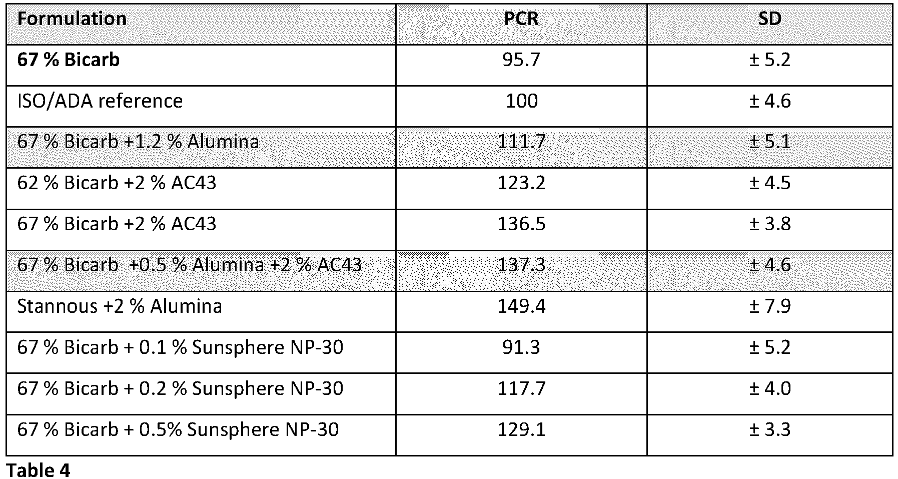

### Testing methods

**Pellicle Cleaning Ratio Test (PCR) as a Measure of Stained Pellicle Removal from Bovine Enamel**

### Introduction

Previous studies (J. Dent. Res., 61:1236, 1982) have indicated that the results of the *in-vitro* PCR test with dentifrice slurries may be considered to be predictive of clinical findings with a reasonable degree of confidence.

Dentifrice formulations were assessed for their ability to remove pellicle stain from bovine enamel and compared with similar comparative formulations (not encompassed within the scope of the invention) containing alternative silica abrasive materials.

### Methodology

Bovine, permanent, central incisors were cut to obtain labial enamel specimens approximately 8×8mm². The enamel specimens were then embedded in an autopolymerizing methacrylate resin so that only the enamel surfaces were exposed. The enamel surfaces were then smoothed on a lapidary wheel and polished with flour of pumice and water, then sonicated to remove excess debris. They were then lightly etched (60 seconds in 0.12M HCl, 30 seconds in saturated NaCO₃ and 60 seconds in 1.0% phytic acid) to expedite stain accumulation and adherence. They were then placed on a rotating rod alternately exposing them to staining broth containing gastric mucin as a protein source and coffee, tea and FeCl₃ 6H₂O as staining sources (i.e., 1.35g coffee, 1.35g tea, 0.02g Fe and 1.0 g mucin in 400ml) for a minimum of 10 days to ensure the specimens developed sufficient stain.

The amount of in vitro stain was graded photometrically (Minolta 2600d, colorimeter) using only the L value of the LAB scale. The area of the specimens measured was a ¼inch diameter circle in the centre of the enamel sample. Specimens with L value measurements between 30-38 (30 being more darkly stained) were used. On the basis of these measurements, the specimens were divided into groups of 16 specimens each, with each group having the same average baseline measurement.

The specimens were then mounted on a mechanical V-8 cross-brushing machine equipped with soft nylon-filament (Oral-B 40 Indicator) toothbrushes. Toothbrushes were conditioned by running the brushing machine for 1,000 strokes in deionised water. Tension on the enamel surface was adjusted to 150g. The dentifrices were used as slurries prepared by mixing 25g of dentifrice with 40ml of deionised water. The ADA reference material was prepared by mixing 10g of material and 50ml of a 0.5% CMC solution. The specimens were brushed for 800 double strokes. To minimise mechanical variables, one specimen per group was brushed on each of the eight brushing heads. Fresh slurries were used for each specimen brushed. Following brushing, specimens were rinsed, blotted dry, and measured again for stain as previously described.

The difference between the pre- and post-brushing stain measurements was determined and the mean and standard error calculated for the reference group. The cleaning ratio for the reference material group was assigned a value of 100. The mean decrement for the reference group was divided into 100 to obtain a constant value to multiple times each individual test decrement within the study. The individual cleaning ratio of each specimen was then calculated (decrement X constant). The mean and standard error mean (SEM) for each group (N=16) was then calculated using the individual cleaning ratios. The larger the value of the cleaning ratio, the greater the amount of stained pellicle removed.

Statistical analyses of the individual means were performed with a one-way analysis of variance (ANOVA) model using Sigma Stat (3.1) Software. Since the ANOVA indicates significant differences, the individual means were analysed by the Student Newman-Keuls (SNK) test.

### RDA: Relative Dentine Abrasivity

The most industry standard way of measuring how abrasive a toothpaste is. This procedure is described in ISO 11609 and ANSI/ADA Standard No. 130.

Each test is a result that is relative to the control and the lower the better.

Dentifrice formulations were assessed for their abrasivity (as determined by RDA) and compared as against similar comparative formulations

### Methodology

The procedure used was the ISO/ADA recommended procedure for determination of dentifrice abrasivity. The dentin specimens (8) were placed in a neutron flux under the controlled conditions outlined by the ISO/ADA. The specimens were then mounted in methyl methacrylate so they would fit in a V-8 cross-brushing machine. The specimens were brushed for 1,500 strokes in a precondition run (since the teeth had been used previously) using a slurry consisting of 10g ISO/ADA reference material in 50ml of a 0.5% CMC glycerin solution. The brushes used were those specified by the ISO/ADA and had been used in a previous study. The brush tension was 150g. Following the precondition run, the test was performed using 150g and 1,500 strokes in a sandwich design in which each test material slurry (25g dentifrice/40ml water) was flanked by reference material slurries (10g ADA reference/50 ml 0.5% CMC).

A 1ml sample was removed from each reference material slurry, weighed (0.01g) and added to 4.5ml of scintillation cocktail. The samples were well mixed and immediately placed on the scintillation counter for radiation detection. Following counting, the net counts per minute (CPM) values were divided by the weight of the sample to calculate the net CPM/gram of slurry. The net CPM/g of the pre- and post-ADA reference material for each test slurry were then calculated and averaged to use in the calculation of the RDA for the test material. The ISO/ADA material was assigned a value of 100 and its ratio to the test material was calculated.

Statistical analyses were performed by a one-way ANOVA model using Sigma Stat Software (13.0). Since significant differences were indicated, the individual means were analysed by the Student Newman Keuls (SNK) test.

### Polishing (Gloss)

### Specimen Preparation and Spectral Reflectance Assessments

Bovine permanent, central incisors (N=8) are cut to obtain labial enamel specimens approximately 10 × 10 mm².

The enamel specimens are then embedded in an autopolymerizing methacrylate resin so that only the enamel surfaces are exposed. The enamel surfaces are then smoothed and polished on a lapidary wheel. The bovine samples are then prescreened by polishing them with a water slurry of diamond abrasive (Buehler, Momocrystalline Diamond Suspension, 3µm and 1µm) to a high luster.

The samples are then scored for surface gloss (gloss units - GU) using a Konica Minolta CM-26dG Spectrophotometer/Gloss Meter. A gloss score of ^{~}100 must be achieved before any specimen is accepted. This procedure is used to confirm the ability of the specimens to achieve a high polish. Two (2) readings per specimen will be taken (90° rotation between measurements) and the mean determined. The specimens will then be etched by decalcifying them in 1% HCL (v/v) for 2 minutes to provide a dull surface to initiate the study. The subsequent gloss reading will be approx. ≤ 15.

### Test Procedure

Following the baseline (etched) scoring, the tooth specimens are placed on a V-8 Cross-Brushing machine. The brush tension are adjusted to 150 grams, and the specimens brushed for 5000 strokes with the appropriate dentifrice slurry (25.0 g of dentifrice + 40.0 g of deionized water) using the ISO/ADA specified toothbrushes (Oral-B, P40, Procter and Gamble, Cincinnati, OH, USA). The specimens are then removed from the brushing machine, rinsed and scored once again for surface gloss (GU) as described above.

### Calculations and Statistical Analyses

The difference between the gloss score (GU) of the etched enamel specimens and the post-treatment score will be calculated for each specimen and will indicate the ability of that treatment to increase the enamel surface gloss/luster/polish. The mean (N=8), standard deviation (SD) and standard error (SEM) will be calculated for each group. Statistical analyses will be conducted by a one-way analysis of variance (ANOVA) / Student-Newman-Keuls (SNK) post-hoc pairwise analysis model using Sigma Plot (14.5) software. Significance of all analyses will be determined at P < 0.050.

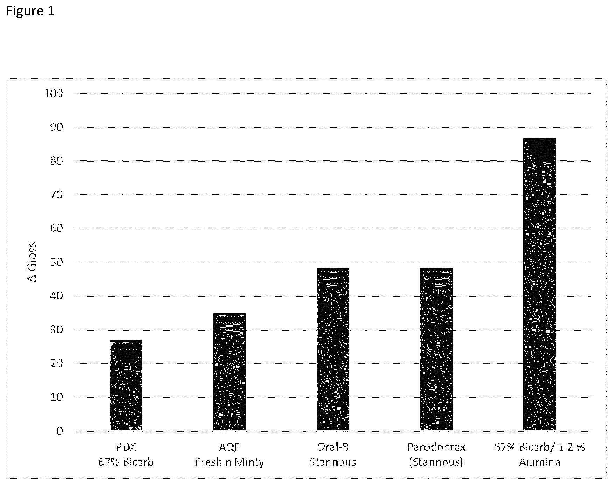

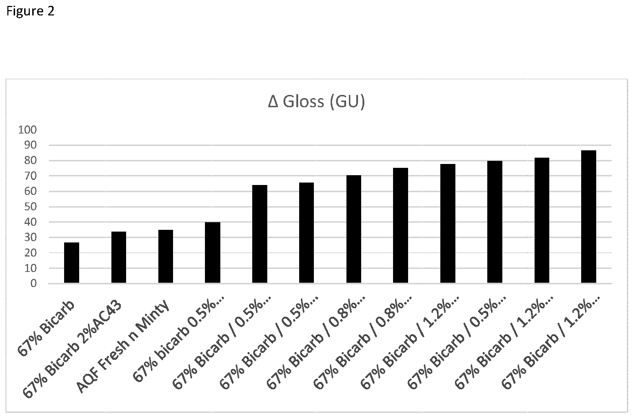

## Claims

1. A dentifrice composition comprising at least about 50 % by weight of sodium bicarbonate and alumina.

2. The dentifrice composition of claim 1 wherein the alumina comprises between about 0.5 and about 3 % by weight.

3. The dentifrice composition according to either claim 1 or claim 2 wherein the alumina comprises between about 1.0 and about 1.5% by weight.

4. The dentifrice composition according to any of the prevision claims wherein the alumina has a d50 particle size of between about 4 µm and 10 µm.

5. The composition according to any one of claims 1 to 4 wherein the sodium bicarbonate is present in an amount from 55 % to 75 % by weight of the composition.

6. The composition according to any of the previous claims wherein the composition comprises between 10 % to 20 % by weight of water.

7. The composition according to any one of the previous claims wherein the composition further comprises a humectant between 5 % and 10 % by weight.

8. The composition according to any one of the previous claims wherein the composition comprises at least one surfactant.

9. The composition according to any one of claims 1 to 8 additionally comprising a fluoride source, a desensitizing agent, an anti-plaque agent, an anti-calculus agent, a whitening agent, an oral malodour agent, an anti-inflammatory agent, an antimicrobial agent, an anti-oxidant, an anti-fungal agent, a wound healing agent or a mixture of at least two thereof.

10. The composition according to any of the previous claims comprising a sweetener, a colourant, a flavouring, a pH modifier, a thickening gum or a mixture of at least two thereof.

11. The dentifrice composition according to any of the previous claims wherein the composition comprises:
Sodium bicarbonate between about 60 % and about 70 % by weight;
Water between about 12 % and about 25 % by weight;
Humectant between about 5 % and about 10 % by weight;
At least one surfactant between about 0.5 % and about 3% by weight; and
Alumina between about 0.5 % and about 3.0 % by weight.

12. The dentifrice composition according to any of the previous claims wherein the composition further comprises an additional abrasive.

13. The dentifrice composition according to any of the previous claims wherein the composition further comprises a spherical silica.

14. The dentifrice composition according to any of the previous claims wherein the composition comprises:
Sodium bicarbonate between about 62 % and about 68 % by weight;
Water between about 15 % and about 22 % by weight;
Glycerol between about 5 % and about 10 % by weight;
At least one surfactant between about 1.0 % and about 2.0 % by weight;
Alumina between about 1.0 % and about 1.5 % by weight; and
A source of fluoride ions.
